# EUROPÄISCHE PATENTANMELDUNG

(11) **EP 4 702 993 A1**
(43) Veröffentlichungstag der Anmeldung: **04.03.2026**
(21) Anmeldenummer: 24197675.2
(22) Anmeldetag: 30.08.2024
(51) Int. Cl.: A61L 2/07, A61L 2/20, A61L 2/26, B25J 1/02

(54) **TRANSFERSYSTEM MIT WENIGSTENS EINER ERSTEN TRANSFERSCHNITTSTELLE EINER ERSTEN KONTROLLIERTEN UMGEBUNG, VERFAHREN ZUM KOPPELN WENIGSTENS EINER ERSTEN TRANSFERSCHNITTSTELLE EINER KONTROLLIERTEN UMGEBUNG MIT WENIGSTENS EINER ZWEITEN TRANSFERSCHNITTSTELLE, VERFAHREN ZUM TRANSFERIEREN EINES GEGENSTANDS**

(71) Anmelder: SKAN AG, 4123 Allschwil (CH)
(72) Erfinder: Latuska, Florian, 4058 Basel (CH); Sommerhalder, Matthias, 4058 Basel (CH); Lehmann, Frank, 4102 Binningen (CH)
(74) Vertreter: Mertzlufft-Paufler, Cornelius

(57) **Zusammenfassung**

Die Erfindung schlägt allgemein ein Transfersystem zum Transferieren von wenigstens einem Gegenstand mit einer Zuführeinrichtung, die von einer ersten kontrollierten Umgebung, insbesondere einem Autoklav, zu einer zweiten kontrollierten Umgebung, insbesondere einem Isolator, über von der ersten und/oder zweiten kontrollierten Umgebung gebildeten, unrunden, insbesondere rechteckigen, Transferschnittstelle beweglich ist, vor, wobei wenigstens zwei Transferschnittstellen derart miteinander koppelbar sind, dass zwischen den Transferschnittstellen ein Zwischenraum und/oder ein Zwischenbereich gebildet ist, wobei der Gegenstand nach einer Dekontamination und/oder Sterilisation der ersten und/oder zweiten kontrollierten Umgebung transferiert wird.

## Beschreibung

Die Erfindung betrifft ein Transfersystem mit wenigstens einer ersten Transferschnittstelle einer ersten kontrollierten Umgebung, die mit wenigstens einer zweiten Transferschnittstelle wenigstens einer zweiten kontrollierten Umgebung koppelbar ist, und einer über die Transferschnittstellen von der ersten kontrollierten Umgebung zu der zweiten kontrollierten Umgebung bewegbaren Zuführeinrichtung.

Die Erfindung betrifft weiter ein Verfahren zum Transferieren eines Gegenstandes, insbesondere von Funktionseinheiten, zwischen wenigstens einer ersten kontrollierten Umgebung und einer zweiten kontrollierten Umgebung, wobei die erste kontrollierte Umgebung und die zweite kontrollierte Umgebung miteinander gekoppelt werden, wobei zunächst die erste und die zweite kontrollierte Umgebung dekontaminiert und/oder sterilisiert werden.

Die Erfindung betrifft ferner ein Verfahren zum Koppeln wenigstens einer ersten Transferschnittstelle einer ersten kontrollierten Umgebung mit wenigstens einer zweiten Transferschnittstelle wenigstens einer zweiten kontrollierten Umgebung mit einem Transfersystem.

Die Erfindung betrifft schließlich ein Verfahren zum Transferieren eines Gegenstands, insbesondere von Funktionseinheiten, zwischen wenigstens einer ersten kontrollierten Umgebung und einer zweiten kontrollierten Umgebung, wobei die erste kontrollierte Umgebung und die zweite kontrollierte Umgebung über einen Zwischenraum miteinander koppelbar sind.

Der Erfindung liegt die Aufgabe zugrunde, einen optimierten und platzsparenden Transfer von Gegenständen, insbesondere Funktionseinheiten, größenunabhängig von einer ersten kontrollierten Umgebung, beispielsweise einem Autoklav, zu einer zweiten kontrollierten Umgebung, beispielsweise einem Isolator, zu ermöglichen und gleichzeitig das Kontaminationsrisiko der zu transferierenden Gegenstände, insbesondere Funktionseinheiten, durch geschickte Kopplung von Transferschnittstellen kontrollierter Umgebungen drastisch zu senken.

Transfersysteme mit miteinander koppelbaren, runden Transferschnittstellen sind aus der Praxis bekannt, beispielsweise als Rapid Transfer Ports (RTP).

Zur Lösung der genannten Aufgabe sind erfindungsgemäß die Merkmale des Anspruchs 1 vorgesehen. Insbesondere wird somit zur Lösung der genannten Aufgabe bei einem Transfersystem der eingangs beschriebenen Art erfindungsgemäß vorgeschlagen, dass die Transferschnittstellen einen unrunden Querschnitt aufweisen.

Eine kontrollierte Umgebung kann sich im Allgemeinen beispielsweise dadurch kennzeichnen, dass diese Fluiddicht von einem äußeren Bereich abgrenzbar ist, um beispielsweise innerhalb der kontrollierten Umgebung positionierte Gegenstände, beispielsweise pharmazeutische Funktionseinheiten, kontaminationsfrei zu präparieren, zu verarbeiten und/oder zu transferieren. Ein äußerer Bereich kann beispielsweise die Umgebungsluft sein.

Die Zuführeinrichtung kann dabei beispielsweise dazu ausgebildet sein, zu transferierende Gegenstände, beispielsweise Funktionseinheiten, über die Systemgrenzen der ersten kontrollierten Umgebung hinweg und zu der zweiten kontrollierten Umgebung zu bewegen, beispielsweise zu verfahren. Hierunter kann auch ein Bewegen in beide Richtungen verstanden werden.

Wie eingangs bereits beschrieben, weisen die Transferschnittstellen der aus der Praxis bekannten Transfersysteme grundsätzlich runde Querschnitte auf.

Eine Problematik von runden Querschnitten der Transferschnittstellen besteht insbesondere darin, dass sich der für den Transfer von Gegenständen, beispielsweise Funktionseinheiten, verfügbare Raum nur dann optimal ausnutzen lässt, wenn die Zuführeinrichtung und/oder beispielsweise die zu transferierenden Gegenstände ebenfalls rund oder gerade so dimensioniert sind, dass diese durch die runden Querschnitte der Transferschnittstellen bewegt werden können. Oftmals besteht die Problematik, dass die zu transferierenden Gegenstände zu groß sind, um diese durch einen runden Querschnitt der Transferschnittstellen, beispielsweise eines weiter oben bereits definierten RTP's, hindurch zu schleußen. Ferner verlangt die Fertigung solcher Ports eine Limitierung der Querschnittgröße.

Unrunde Querschnitte, insbesondere unrunde Öffnungsquerschnitte der Transferschnittstellen haben hingegen den Vorteil, dass mit einer vergleichbaren Größe ein größerer lichter Innenquerschnitt der Transferschnittstellen erreicht werden kann. Somit können die zu transferierenden Gegenstände größer ausgestaltet werden, wodurch weniger Transfervorgänge benötigt werden, um dieselbe Menge an Gegenständen von der ersten kontrollierten Umgebung über die Zuführeinrichtung zur zweiten kontrollierten Umgebung zu transferieren.

Je nach Form der zu transferierenden Gegenstände müssen diese bei runden Querschnitten der Transferschnittstellen zur maximalen Ausnutzung des zur Verfügung stehenden Raumes, zumindest eine Abmessung, insbesondere eine Abmessung senkrecht zu einer horizontalen Bewegungsrichtung dieser Gegenstände, aufweisen, die gerade kleiner ist, als ein Außendurchmesser der runden Transferschnittstellen, sodass diese horizontal mittig durch den Öffnungsquerschnitt der Transferschnittstellen bewegbar sind.

Eine Abweichung von der horizontal mittig ausgerichteten Transferebene und/oder der maximal möglichen Abmessung der zu transferierenden Gegenstände, begrenzt durch die Öffnungsquerschnitte der Transferschnittstellen, würde keinen Transfer der Gegenstände mehr zulassen, da, aufgrund des runden Querschnitts der Transferschnittstellen, die zu transferierenden Gegenstände beispielsweise mit den sich in radialer Richtung verkleinernden Kreissektoren des Öffnungsquerschnittes der Transferschnittstellen kollidieren würden. Eine Abweichung von der horizontal mittig ausgerichteten Transferebene wäre lediglich dadurch möglich, wenn die oder eine Abmessung der zu transferierenden Gegenstände nochmals verringert wird, was jedoch ebenfalls zur Folge hätte, dass mehr Transfervorgänge benötigt würden, um dieselbe Menge an Gegenständen von der einen kontrollierten Umgebung zur anderen kontrollierten Umgebung über die Zuführeinrichtung zu transferieren.

Folglich bieten runde Öffnungsquerschnitte der Transferschnittstellen nicht nur Abschnitte, die nicht aktiv zum Transfer von Gegenständen genutzt werden können, sondern auch gleichzeitig eine Angriffsfläche für mikrobiologische Verunreinigungen, die das Risiko einer Kontamination dieser Abschnitte und auch der zu transferierenden Gegenstände erhöhen.

Unrunde Querschnitte der Funktionseinheiten bieten hingegen den weiteren Vorteil, dass die Querschnitte der Transferschnittstellen optimal an die geometrischen Ausgestaltungen der zu transferierenden Gegenstände, beispielsweise Funktionseinheiten, angepasst werden können. Der zum Transferieren der Gegenstände ohnehin nicht genutzte Raum runder Öffnungsquerschnitte der Transferschnittstellen kann deutlich reduziert und das Dekontaminationsrisiko besagter Abschnitte der Transferschnittstellen folglich stark reduziert werden.

Vorzugsweise weisen die Transferschnittstellen dabei einen rechteckigen Querschnitt auf.

Bei einer weiteren vorteilhaften Ausgestaltung der Erfindung kann vorgesehen sein, dass wenigstens eine kontrollierte Umgebung ein Isolator ist.

Isolatoren, beispielsweise auch RABS ("Restricted-accessbarrier-systems") werden in der Industrie genutzt, um beispielsweise pharmazeutische Produkte, die Bestandteil der Funktionseinheiten sein können, steril beziehungsweise keimfrei (weiter)zu verarbeiten.

Der Isolator kann dabei beispielsweise in einem Reinraum aufgestellt sein.

Alternativ oder zusätzlich kann vorgesehen sein, dass die wenigstens eine kontrollierte Umgebung ein Autoklav ist.

Als Autoklav kann beispielsweise ein gasdicht verschließbarer Druckbehälter verstanden werden, in welchem beispielsweise zu transferierende Gegenstände, vorzugsweise Funktionseinheiten, insbesondere durch thermische Behandlung, beispielsweise mittels Heißdampfs, vorzugsweise im Überdruckbereich sterilisiert werden.

Besonders vorteilhaft kann hier sein, wenn die erste kontrollierte Umgebung als Autoklav und die zweite kontrollierte Umgebung als Isolator ausgebildet ist. Ein direkter Transfer der Gegenstände, beispielsweise von Funktionseinheiten, über die Zuführeinrichtung von dem Autoklav in den Isolator kann somit ermöglicht werden.

Bei einer solchen Ausgestaltung der Erfindung ergibt sich der weitere hervorragende Vorteil, dass Autoklav und Isolator nicht nur in einem gemeinsamen Reinraum positioniert werden können, sondern dass Autoklav und Isolator in direkter Weise miteinander koppelbar sind. Der Arbeitsaufwand und das Dekontaminationsrisiko kann im Vergleich zu einer bislang üblichen externen Autoklavierung von Funktionseinheiten deutlich verringert werden.

Alternativ oder zusätzlich kann vorgesehen sein, dass wenigstens eine kontrollierte Umgebung mobil oder stationär ist.

Beispielsweise kann dies bedeuten, dass die erste kontrollierte Umgebung als Autoklav und die zweite kontrollierte Umgebung als Isolator ausgebildet ist, wobei beispielsweise entweder der Autoklav stationär und der Isolator mobil oder der Autoklav mobil und der Isolator stationär ausgebildet sind. Hierdurch ergibt sich der Vorteil, dass beide kontrollierten Umgebungen auch dann miteinander gekoppelt werden können, wenn lediglich eine der kontrollierten Umgebungen mobil ausgebildet ist.

Bei einer weiteren vorteilhaften Ausgestaltung der Erfindung kann vorgesehen sein, dass wenigstens eine kontrollierte Umgebung derart bewegbar ist, dass die Zuführeinrichtung innerhalb der kontrollierten Umgebung von einer Beförderungseinheit transportierbar ist.

Die Beförderungseinheit kann dabei beispielsweise von einem Wagen mit Rädern gebildet sein, über die die kontrollierte Umgebung vorteilhafterweise beliebig bewegt werden kann.

Alternativ oder zusätzlich kann vorgesehen sein, dass wenigstens eine kontrollierte Umgebung derart bewegbar ist, dass die Zuführeinrichtung in einer Orientierung in der kontrollierten Umgebung fixiert ist.

Besonders vorteilhaft kann somit beispielsweise eine gleichbleibende Orientierung der Zuführeinrichtung und der darauf abgelegten Gegenstände, insbesondere der Funktionseinheiten, über vorzugsweise einen Transportweg der kontrollierten Umgebung gewährleistet werden.

Bei einer weiteren vorteilhaften Ausgestaltung der Erfindung kann vorgesehen sein, dass die Transferschnittstellen Türen haben.

Besonders vorteilhaft sind Türen besonders leicht bedienbar und miteinander koppelbar. Beispielsweise kann auch vorgesehen sein, dass die Türen sterilisierbare Dichtungen aufweisen.

Insbesondere sind die Türen druckdicht.

Falls die erste kontrollierte Umgebung beispielsweise als Autoklav und die zweite kontrollierte Umgebung als Isolator ausgebildet ist, ergibt sich durch die druckdichten Türen der besondere Vorteil, dass beide kontrollierten Umgebungen beispielsweise bereits während eines Autoklavierprozesses miteinander koppelbar sind und die Transferschnittstellen somit insbesondere dem Druck während des Autoklavierens standhalten können, sodass der Prozess des Autoklavierens vorzugsweise keinen Einfluss auf den Isolator hat und umgekehrt.

Alternativ oder zusätzlich kann vorgesehen sein, dass ein Zwischenbereich zwischen den Transferschnittstellen, insbesondere den Türen, abdichtbar und/oder evakuierbar ist.

Als Zwischenbereich kann beispielsweise eine schmale Kavität zwischen den wenigstens zwei miteinander gekoppelten Transferschnittstellen, die in gekoppeltem Zustand insbesondere fest aneinander anliegen, angesehen werden.

Unter "evakuierbar" kann allgemein beispielsweise verstanden werden, dass der Zwischenbereich, und/oder, wie weiter unten definiert ein Zwischenraum, von daran angrenzenden Bereichen, beispielsweise den kontrollierten Umgebungen, abgegrenzt werden kann.

Unter "abdichtbar" kann beispielsweise verstanden werden, dass, die Räume zwischen den Transferschnittstellen derart abgedichtet werden können, dass kein Fluidaustausch mit daran angrenzenden Bereichen, beispielsweise den kontrollierten Umgebungen, erfolgt. Dies kann beispielsweise durch eine Dichtung realisiert werden. Die Dichtung kann dabei beispielsweise ebenfalls unrund, insbesondere rechteckig, sein und/oder sich an eine Form der Transferschnittstelle anpassen. Die Dichtung kann dabei oder allgemein beispielsweise aufblasbar sein.

Insbesondere sind die Türen bei evakuiertem und/oder abgedichteten Zwischenbereich miteinander beweglich.

Hierdurch ergibt sich insbesondere der hervorragende Vorteil, dass ein Öffnen und/oder Schließen und somit ein Abgrenzen der über die Türen miteinander koppelbaren kontrollierten Umgebungen schnell und einfach erfolgen kann.

Besonders vorteilhaft kann somit beispielsweise ebenso auf ein Twist-Lock-System, das bei den bekannten RTP's genutzt wird, verzichtet werden, um beispielsweise ein Kontaminationsrisiko der Transferschnittstellen zu minimieren.

Alternativ oder zusätzlich kann vorgesehen sein, dass ein Zwischenraum zwischen den Transferschnittstellen, insbesondere den Türen, abdichtbar und/oder evakuierbar ist.

Als Zwischenraum kann hierbei beispielsweise ein Raum zwischen zwei voneinander beabstandeten Transferschnittstellen verstanden werden. Dieser Raum kann beispielsweise vorteilhaft dazu genutzt werden, die Transferschnittstellen derart zu öffnen und/oder zu schließen, dass diese nicht in die eine kontrollierte Umgebung und/oder die andere kontrollierte Umgebung ragen, sodass beispielsweise anstelle einer Klappbewegung der Transferschnittstellen während des Öffnens und/oder Schließens in Richtung des Zwischenraumes und nicht in Richtung oder in eine/r der kontrollierten Umgebungen erfolgt. Ferner kann beispielsweise vorgesehen sein, dass die Transferschnittstellen zum Öffnen und/oder Schließen in einem im Zwischenraum vorgesehenen Bereich positioniert werden, beispielsweise unterhalb der Querschnitte der Transferschnittstellen.

Hierbei kann beispielsweise ebenfalls vorgesehen sein, dass eine der wenigstens zwei kontrollierten Umgebungen zwei voneinander beabstandete Transferschnittstellen ausbildet, wobei die jeweils andere kontrollierte Umgebung eine weitere Transferschnittstelle ausbildet. Wird beispielsweise die eine Transferschnittstelle der einen kontrollierten Umgebung, die wiederum beabstandet ist von einer weiteren Transferschnittstelle derselben kontrollierten Umgebung mit der Transferschnittstelle der weiteren kontrollierten Umgebung gekoppelt, bildet sich zwischen der Transferschnittstelle der einen kontrollierten Umgebung und der Transferschnittstelle der weiteren kontrollierten Umgebung ein abdichtbarer und/oder evakuierbarer Zwischenraum. In diesem Fall wären somit wenigstens drei Transferschnittstellen ausgebildet, wobei zwei Transferschnittstellen direkt an eine äußere Umgebung angrenzen. Ein besonderer Vorteil ergibt sich hierbei dadurch, dass mögliche, insbesondere mikrobiologische, Verunreinigungen in diesem Zwischenraum zu keiner Kontamination der damit gekoppelten kontrollierten Umgebung(en) und umgekehrt führt.

Ferner kann der Zwischenraum und/oder der Zwischenbereich beispielsweise unabhängig von den kontrollierten Umgebungen dekontaminiert werden.

Beispielsweise kann auch vorgesehen sein, dass der Zwischenraum als adaptierbare Einheit mobil zwischen die kontrollierten Umgebungen schaltbar oder stationär mit einer der kontrollierten Umgebungen gekoppelt ist, wie weiter oben beschrieben.

Bei einer weiteren vorteilhaften Ausgestaltung kann vorgesehen sein, dass das Transfersystem ein Rapid Transfer Port (RTP) ist.

Besonders vorteilhaft können somit die bekannten Vorteile des Rapid Transfer Port mit den bereits erwähnten Vorteilen kombiniert werden.

Bei einer weiteren vorteilhaften Ausgestaltung kann vorgesehen sein, dass die Transferschnittstellen kraft- und/oder formschlüssig miteinander koppelbar sind.

Als kraft- und/oder formschlüssige Verbindungen kommen sämtliche bekannten Kraft- und/oder Formschlussverbindungen in Frage. Vorteilhaft kann somit ein Abdichten und/oder Evakuieren der Transferschnittstellen erreicht werden.

Bei einer weiteren vorteilhaften Ausgestaltung der Erfindung kann vorgesehen sein, dass die Transferschnittstellen der kontrollierten Umgebungen jeweils eine dichte Begrenzung der kontrollierten Umgebung bilden.

Eine dichte Begrenzung kann beispielsweise eine Außenwand der kontrollierten Umgebung sein.

Bei einer weiteren vorteilhaften Ausgestaltung kann vorgesehen sein, dass in der ersten kontrollierten Umgebung wenigstens eine Funktionseinheit und in der zweiten kontrollierten Umgebung wenigstens eine Verarbeitungsstation für Verbrauchsmaterial aufgenommen ist.

Besonders vorteilhaft kann die Funktionseinheit somit problemlos von der Zuführeinrichtung von der einen kontrollierten Umgebung, beispielsweise einem Autoklav, bis zu der Verarbeitungsstation bewegt werden.

Als Funktionseinheit können verschiedene in einer kontrollierten Umgebung, beispielsweise einem Isolator, zu verarbeitende und/oder darin zu verbauende Einheiten verstanden werden, die beispielsweise vor einem Einbau in den oder einen Isolator autoklaviert werden müssen, um verschiedene Anforderungen, beispielsweise an Sterilität, zu erlangen.

Allgemein kann gesagt werden, dass es sich bei den Funktionseinheiten um Einheiten handelt, die entweder direkt oder indirekt mit Teilen in Kontakt kommen, die vor einer Dekontamination bewahrt werden müssen, wie beispielsweise Medikamente aufnehmende Behältnisse, Spritzen, Stopfen und/oder Kappen et cetera.

Einige Beispiele hierfür können Führungsmittel oder Sortiermittel für Verschlusselemente sein, die in der pharmazeutischen Verarbeitung beispielsweise zum Verschließen der Behältnisse, wie beispielsweise Stopfen, Kappen und/oder Plunger, um nur einige zu nennen, genutzt werden, oder aber auch verschiedenste Aufbauten wie beispielsweise Schienen, die in kontrollierten Umgebungen aufgebaut werden, um insbesondere verschiede Transportvorgänge der Teile ermöglichen zu können.

Insbesondere kann es sich bei der Funktionseinheit um eine Bevorratungseinheit für Verbrauchsmaterial handeln.

Die Bevorratungseinheit kann dabei beispielsweise ein Topf, insbesondere ein Rütteltopf, sein, in welchem das Verbrauchsmaterial, beispielsweise Stopfen, Kappen und/oder Plunger, bevorratet ist. Weitere Verbrauchsmaterialien, die in einer Bevorratungseinheit aufgenommen werden können, sind möglich.

Hierdurch ergibt sich beispielsweise die hervorragende Möglichkeit, dass, angenommen, die erste kontrollierte Umgebung ist als Autoklav und die zweite kontrollierte Umgebung als Isolator ausgebildet, die Funktionseinheiten von der Zuführeinrichtung direkt nach dem Autoklovieren von dem Autoklaven durch Öffnen der Transferschnittstellen, die Autoklav und Isolator voneinander abgrenzen, nicht nur zum Isolator, sondern sogar direkt zu der oder einer Verarbeitungsstation im Isolator bewegt werden können.

Als Verarbeitungsstation können beispielsweise die das Verarbeitungsmaterial zu verarbeitenden Stationen betrachtet werden, wie beispielsweise Greifen eines Stopfens, Aufsetzen auf ein Behältnis, Verschließen et cetera.

Alternativ oder zusätzlich sind die Merkmale auf ein Transfersystem nach dem Oberbegriff von Anspruch 1 oder auf das bereits beanspruchte Transfersystem vorgesehen. Insbesondere wird somit erfindungsgemäß vorgeschlagen, dass die Transferschnittstellen den oder einen Zwischenbereich und/oder den oder einen Zwischenraum begrenzen, in dem eine dritte kontrollierte Umgebung gebildet ist.

Besonders vorteilhaft kann der Zwischenbereich, beispielsweise der bereits beschriebene Zwischenbereich und/oder der Zwischenraum, beispielsweise der bereits beschriebene Zwischenraum, unabhängig von den anderen kontrollierten Umgebungen vorbehandelt, insbesondere dekontaminiert, werden. Ein weiterer Vorteil kann dabei darin bestehen, dass das Risiko gesenkt werden kann, dass Verunreinigungen, insbesondere mikrobiologische Verunreinigungen, von dem Zwischenraum und/oder dem Zwischenbereich an die angrenzenden kontrollierten Umgebungen übertragen werden und auch umgekehrt. Es kann somit beispielsweise auch verhindert werden, dass der Zwischenraum und/oder der Zwischenbereich durch die eingangs beschriebene Umgebungsluft dekontaminiert wird. Falls eine Dekontamination dennoch eintritt, beispielsweise sobald der Zwischenraum und/oder der Zwischenbereich beispielsweise nach außen geöffnet und der Umgebungsluft präsentiert wird, kann vorzugsweise ein Dekontaminationsvorgang des Zwischenraums und/oder des Zwischenbereichs, wie weiter unten noch näher spezifiziert, initiiert werden.

Bei einer weiteren vorteilhaften Ausgestaltung kann vorgesehen sein, dass der Zwischenbereich und/oder der Zwischenraum abgedichtet und/oder evakuiert ist.

Besonders vorteilhaft können die Transferschnittstellen somit beispielsweise gemeinsam geöffnet und/oder geschlossen werden. Ferner kann beispielsweise verhindert werden, dass Verunreinigungen in den Zwischenraum und/oder Zwischenbereich geraten oder aus dem Zwischenraum und/oder Zwischenbereich heraustreten.

Das Abdichten und/oder Evakuieren kann beispielsweise durch Vakuumerzeugung im Zwischenbereich und/oder im Zwischenraum erfolgen.

Alternativ oder zusätzlich kann vorgesehen sein, dass der Zwischenbereich mit Türen der Transferschnittstellen mitbewegbar ist.

Besonders vorteilhaft kann somit ebenfalls das Dekontaminationsrisiko nochmals drastisch gesenkt werden.

Bei einer weiteren vorteilhaften Ausgestaltung kann die Erfindung vorsehen, dass die Transferschnittstellen miteinander koppelbar sind, insbesondere zum gemeinsamen Öffnen und/oder Schließen.

Hierdurch ergibt sich beispielsweise der Vorteil, dass das Bewegen der Zuführeinrichtung von der ersten kontrollierten Umgebung zur zweiten kontrollierten Umgebung (und umgekehrt) problemlos realisiert und vereinfacht werden kann.

Bei einer weiteren vorteilhaften Ausgestaltung kann vorgesehen sein, dass wenigstens eine Transferschnittstelle von einem Blinddeckel gebildet ist.

Dabei kann beispielsweise vorgesehen sein, dass der Blinddeckel eine Transferschnittstelle des Zwischenraums bildet, die an die Umgebung angrenzt.

Der Blinddeckel kann dabei beispielsweise manuell abnehmbar oder aber eine maschinell verfahrbare Tür sein.

Bei einer weiteren vorteilhaften Ausgestaltung der Erfindung kann vorgesehen sein, dass ein Volumen des Zwischenraums und/oder des Zwischenbereichs kleiner ist als ein Volumen der ersten und/oder der zweiten kontrollierten Umgebung.

Besonders vorteilhaft kann somit beispielsweise erreicht werden, dass nach dem Koppeln der Transferschnittstellen nicht die erste und zweite kontrollierte Umgebung mit größerem Volumen, sondern lediglich der Zwischenraum mit dem kleineren Volumen dekontaminiert und/oder sterilisiert werden muss, ohne dass ein vergrößertes Dekontaminationsrisiko besteht.

Alternativ oder zusätzlich sind zur Lösung der genannten Aufgabe erfindungsgemäß die Merkmale des nebengeordneten, auf ein Verfahren gerichteten Anspruchs vorgesehen. Insbesondere wird somit zur Lösung der genannten Aufgabe bei einem Verfahren der eingangs beschriebenen Art erfindungsgemäß vorgeschlagen, dass nach der Dekontamination und/oder Sterilisation eine Zuführeinrichtung eines Transfersystems, von der ersten kontrollierten Umgebung zur zweiten kontrollierten Umgebung bewegt wird.

Besonders vorteilhaft kann somit insbesondere verhindert werden, dass Verunreinigungen, insbesondere mikrobiologische Verunreinigungen, von der ersten kontrollierten Umgebung zur zweiten kontrollierten Umgebung übertragen werden. Dabei kann beispielsweise vorgesehen sein, dass die erste kontrollierte Umgebung ein Autoklav und die zweite kontrollierte Umgebung ein Isolator ist.

Beispielsweise kann vorgesehen sein, dass der Gegenstand zunächst im Autoklav sterilisiert wird. Bevor der Gegenstand jedoch von dem Autoklav zum Isolator über die Zuführeinrichtung bewegt werden kann, kann vorgesehen sein, dass der Isolator zunächst dekontaminiert wird, beispielsweise mit Wasserstoffperoxid (H₂O₂).

Insbesondere handelt es sich bei dem Transfersystem um ein bereits beanspruchtes Transfersystem.

Somit können insbesondere die bereits beschriebenen Vorteile realisiert werden.

Bei einer weiteren vorteilhaften Ausgestaltung der Erfindung kann vorgesehen sein, dass die Transferschnittstelle, insbesondere durch eine Klappbewegung, in einen Bereich bewegt wird, der außerhalb einer Transferbewegung der Zuführeinrichtung liegt, wobei der Bereich ebenfalls dekontaminiert wird.

Besonders vorteilhaft kann somit beispielsweise die gesamte Transferschnittstelle, insbesondere mitsamt einer diese aufweisenden Dichtung, dekontaminiert werden. Die Dichtung kann dabei beispielsweise unrund, insbesondere rechteckig, sein und/oder sich an eine Form der Transferschnittstelle anpassen. Die Dichtung kann dabei oder allgemein beispielsweise aufblasbar sein.

Alternativ oder zusätzlich sind zur Lösung der genannten Aufgabe erfindungsgemäß die Merkmale des nebengeordneten, auf ein Verfahren gerichteten Anspruchs vorgesehen. Insbesondere wird somit zur Lösung der genannten Aufgabe bei einem Verfahren der eingangs beschriebenen Art erfindungsgemäß vorgeschlagen, dass die Transferschnittstellen einen Zwischenraum und/oder einen Zwischenbereich begrenzen, der vor einem Öffnen wenigstens einer Transferschnittstelle wenigstens einer kontrollierten Umgebung abgedichtet und/oder evakuiert wird.

Der Zwischenraum und/oder der Zwischenbereich kann beispielsweise ein weiter oben bereits definierter Zwischenraum und/oder Zwischenbereich sein.

Das Öffnen der Transferschnittstelle kann beispielsweise dann erfolgen, wenn die kontrollierten Umgebungen miteinander gekoppelt werden. Da dies ein hinsichtlich Dekontaminationsübertragung kritischer Moment ist, kann durch Abdichtung und/oder Evakuierung des Zwischenbereichs und/oder des Zwischenraums verhindert werden, dass Verunreinigungen, beispielsweise mikrobiologische Verunreinigungen, von dem Zwischenbereich und/oder dem Zwischenraum an die kontrollierten Umgebungen übertragen wird oder umgekehrt.

Bei einer weiteren vorteilhaften Ausgestaltung der Erfindung kann vorgesehen sein, dass vor einer Dekontamination des Zwischenraums und/oder des Zwischenbereichs wenigstens eine kontrollierte Umgebung sterilisiert und/oder dekontaminiert wird.

Beispielsweise kann dabei vorgesehen sein, dass die erste kontrollierte Umgebung ein Autoklav, beispielsweise der bereits beschriebene Autoklav, und die zweite kontrollierte Umgebung ein Isolator, beispielsweise der bereits beschriebene Isolator, ist, wobei beide kontrollierte Umgebungen über die Transferschnittstellen, die den Zwischenraum und/oder den Zwischenbereich begrenzen, miteinander gekoppelt sind.

Um die kontrollierte Umgebung, beispielsweise den Isolator und/oder den Autoklaven, vor Verunreinigungen, insbesondere mikrobiologischen Verunreinigungen, die wiederum zu einer Kontamination darin verarbeiteter und/oder aufgebauter Funktionseinheiten, beispielsweise bereits erwähnten und/oder beanspruchten Funktionseinheiten, führen, zu schützen, wird innerhalb einer der, oder auch beiden, kontrollierten Umgebungen zunächst ein Dekontaminationsvorgang und/oder ein Sterilisationsvorgang durchgeführt.

Im Rahmen der Dekontamination wird die wenigstens eine kontrollierte Umgebung, im oben bezeichneten Beispiel der Isolator, vorzugsweise mit Wasserstoffperoxid (H₂O₂) durchströmt.

Bei der Sterilisation wird beispielsweise der Autoklav mittels Heißdampf unter hohen Druck versetzt.

Alternativ oder zusätzlich kann auch vorgesehen sein, dass während der Dekontamination und/oder Sterilisation des Zwischenraums und/oder des Zwischenbereichs wenigstens eine kontrollierte Umgebung sterilisiert und/oder dekontaminiert wird.

Hierdurch kann beispielsweise vorteilhaft ein verschnellerter Dekontaminations- und/oder Sterilisationsvorgang gewährleistet werden, da der Zwischenraum und/oder der Zwischenbereich zeitgleich mit wenigstens einer kontrollierten Umgebung sterilisiert und/oder dekontaminiert werden kann.

Bei einer weiteren vorteilhaften Ausgestaltung der Erfindung kann vorgesehen sein, dass der Zwischenraum und/oder der Zwischenbereich während der Dekontamination und/oder der Sterilisation abgedichtet und/oder evakuiert wird. Vorteilhaft ist hierbei, dass der Zwischenraum und/oder der Zwischenbereich somit unabhängig von der wenigstens einen kontrollierten Umgebung dekontaminiert und/oder sterilisiert werden können.

Eine weitere vorteilhafte Ausgestaltung der Erfindung kann vorsehen, dass die Transferschnittstellen von, insbesondere druckdichten, Türen gebildet sind, die vor, nach oder während der Dekontamination und/oder Sterilisation wenigstens einer kontrollierten Umgebung geöffnet werden.

Werden die, insbesondere druckdichten, Türen vor der Dekontamination und/oder Sterilisation wenigstens einer kontrollierten Umgebung geöffnet, kann beispielsweise vorteilhaft sein, wenn die Türen den oder einen Zwischenraum begrenzen, sodass dieser zeitgleich mit der kontrollierten Umgebung dekontaminiert und/oder sterilisiert werden kann.

Sobald die von dem Zwischenraum an die kontrollierte Umgebung angrenzenden Türen geöffnet und die von den direkt an die kontrollierte Umgebung angrenzenden Türen beabstandeten Türen, die hierbei insbesondere eine dichte Begrenzung zur Umgebung ausbilden, geschlossen sind, kann die kontrollierte Umgebung vorzugsweise zeitgleich mit dem Zwischenraum dekontaminiert und/oder sterilisiert werden. Hierbei kann beispielsweise auch vorgesehen sein, dass die an die Umgebung angrenzenden Türen evakuiert und/oder abgedichtet sind, die geöffneten Türen hingegen nicht, sodass lediglich an den Türen, die die dichte Begrenzung bilden, einen Zwischenbereich ausgebildet ist. Dies ist jedoch nicht zwingend notwendig, es kann ferner möglich sein, dass auch die an die kontrollierte Umgebung angrenzenden Türen einen abgedichteten und/oder evakuierten Zwischenbereich bilden, der separat dekontaminiert und/oder sterilisiert wird.

Besonders vorteilhaft ist hierbei, wenn die geöffnete Transferschnittstellen, beziehungsweise Türen, innerhalb des Dekontaminationsbereichs und/oder Sterilisationsbereichs der kontrollierten Umgebung und/oder des Zwischenbereichs positioniert werden kann. Eine weitere Möglichkeit könnte auch beispielsweise darin bestehen, dass die geöffneten Türen in einem Bereich innerhalb der kontrollierten Umgebung positioniert werden, in dem eine zusätzliche Dekontamination und/oder Sterilisation erfolgt, beispielsweise durch eine in dem Bereich positioniert, zusätzliche Wasserstoffperoxid-Düse.

Es können somit vorteilhafterweise die wenigstens eine kontrollierte Umgebung, der Zwischenbereich und wenigstens eine Transferschnittstelle, in diesem Falle wenigstens eine Türe, gemeinsam dekontaminiert und/oder sterilisiert werden.

Dieselben Vorteile ergeben sich beispielsweise auch dann, wenn die Transferschnittstellen während der Dekontamination und/oder Sterilisation wenigstens einer kontrollierten Umgebung geöffnet werden.

Werden die von den Türen gebildeten Transferschnittstellen erst nach der Dekontamination und/oder Sterilisation wenigstens einer kontrollierten Umgebung geöffnet, kann beispielsweise vorgesehen sein, dass, falls ein Zwischenraum zwischen den Türen vorhanden ist, dieser vor der Dekontamination und/oder Sterilisation der kontrollierten Umgebung dekontaminiert und/oder sterilisiert wird.

Vorzugsweise werden die Türen dabei durch eine Klappbewegung und/oder eine Schiebebewegung geöffnet. Hierdurch können vorteilhafterweise verschiedene, dem Bauraum oder verschiedenen weiteren Bedingungen angepasste Öffnungs- und/oder Schließbewegungen der Türen realisiert werden.

Alternativ oder zusätzlich sind zur Lösung der genannten Aufgabe erfindungsgemäß die Merkmale des nebengeordneten, auf ein Verfahren gerichteten Anspruchs vorgesehen. Insbesondere wird somit zur Lösung der genannten Aufgabe bei einem Verfahren zum Transferieren eines Gegenstands der eingangs beschriebenen Art erfindungsgemäß vorgeschlagen, dass der Zwischenraum vor der Kopplung nach außen offen ist und der Zwischenraum durch eine Begrenzung der ersten kontrollierten Umgebung verschlossen wird, dass der verschlossene Zwischenraum dekontaminiert wird und dass nach der Dekontamination zumindest die erste kontrollierte Umgebung zum Zwischenraum geöffnet wird.

Besonders vorteilhaft kann der Zwischenraum somit vor der Kopplung separat, beispielsweise räumlich getrennt von der den kontrollierten Umgebungen, dekontaminiert werden kann. Vorzugsweise wird die erste kontrollierte Umgebung vor dem Öffnen ebenfalls dekontaminiert und/oder sterilisiert. Dies kann beispielsweise in bereits beschriebener Weise funktionieren. Die zweite kontrollierte Umgebung kann ebenso vor dem Koppeln dekontaminiert und/oder sterilisiert werden. Zumindest sollte eine Dekontamination und/oder Sterilisation der ersten und/oder zweiten kontrollierten Umgebung durchgeführt werden, bevor ein Zugang zum Zwischenraum geöffnet wird.

Bei einer weiteren vorteilhaften Ausgestaltung kann vorgesehen sein, dass die Koppelung über wenigstens eine Transferschnittstelle erfolgt.

Besonders vorteilhaft können somit die bereits beschriebenen Vorteile realisiert werden.

Beispielsweise kann dabei auch vorgesehen sein, dass wenigstens eine Transferschnittstelle, beispielsweise die Transferschnittstelle der ersten und/oder der zweiten kontrollierten Umgebung und/oder des Zwischenraums, kleiner dimensioniert ist als eine Dichtung, die an dem Zwischenraum ausgebildet ist.

Insbesondere sind die Transferschnittstellen von druckdichten Türen gebildet.

Hierdurch ergibt sich, wie bereits beschrieben, beispielsweise der Vorteil, dass diese, falls eine der kontrollierten Umgebungen beispielsweise als Autoklav ausgebildet ist, dem während des Autoklavierens herrschenden Druckes standhalten.

Bei einer weiteren vorteilhaften Ausgestaltung der Erfindung kann vorgesehen sein, dass zum Verschließen und/oder Öffnen des Zwischenraums wenigstens eine Transferschnittstelle, insbesondere druckdichte Tür, derart bewegt wird, dass sich diese, zumindest teilweise, innerhalb des Zwischenraums befindet.

Besonders vorteilhaft kann somit beispielsweise nicht nur die Transferschnittstelle, sondern auch die oder eine diese umgebende Dichtung dekontaminiert werden.

Alternativ oder zusätzlich sind die Merkmale auf ein Transfersystem nach dem Oberbegriff von Anspruch 16 oder auf das bereits beanspruchte Verfahren vorgesehen. Insbesondere wird somit erfindungsgemäß vorgeschlagen, dass zur Dekontamination ein Dekontaminationsmittel in den Zwischenraum unbeigemischt eingebracht und zerstäubt wird.

Als Dekontaminationsmittel kann beispielsweise Wasserstoffperoxid (H₂O₂) eingesetzt werden.

Alternativ oder zusätzlich ist erfindungsgemäß vorgesehen, dass zur Dekontamination ein Dekontaminationsmittel in eine erste und/oder eine zweite kontrollierte Umgebung unbeigemischt eingebracht und zerstäubt wird.

Die Zerstäubung kann dabei beispielsweise mittels einer Düse und/oder beispielsweise durch Ultraschalleinwirkung auf das Dekontaminationsmittel erfolgen. Weitere Zerstäubungsmechanismen sind möglich, auch wenn nicht näher beschrieben.

Die Erfindung wird nun anhand von Ausführungsbeispielen näher beschrieben, ist jedoch nicht auf die Ausführungsbeispiele beschränkt. Weitere Ausführungsbeispiele ergeben sich durch Kombination der Merkmale einzelner oder mehrerer Ansprüche untereinander und/oder mit einzelnen oder mehreren Merkmalen des Ausführungsbeispiels.

Es zeigt, in jeweils stark vereinfachter Darstellung,
- Fig. 1: eine zweidimensionale Prinzipdarstellung eines Verfahrens zum Transferieren von Gegenständen mittels einem Transfersystems mit zwei über eine Transferschnittstelle gekoppelten, kontrollierten Umgebungen und bewegbarer Zuführeinrichtung,
- Fig. 2: eine zweidimensionale Prinzipdarstellung eines Verfahrens zum Transferieren von Gegenständen mittels einem Transfersystem mit einer mobilen kontrollierten Umgebung, die mit einer zweiten kontrollierten Umgebung über zwei miteinander bewegliche Transferschnittstellen koppelbar ist,
- Fig. 3: eine weitere, zweidimensionale Prinzipdarstellung eines Verfahrens zum Transferieren von Gegenständen mittels Transfersystem, wobei die Transferschnittstellen einen Zwischenraum ausbilden, über die die kontrollierten Umgebungen miteinander gekoppelt werden und wobei der Zwischenraum separat dekontaminiert wird,
- Fig. 4: eine weitere, zweidimensionale Prinzipdarstellung eines Verfahrens zum Transferieren von Gegenständen mittels Transfersystem gemäß Fig. 3, wobei der Zwischenraum gemeinsam mit der kontrollierten Umgebung dekontaminiert wird,
- Fig. 5: eine weitere, zweidimensionale Prinzipdarstellung eines Verfahrens zum Transferieren von Gegenständen mittels mehrerer Transfersysteme

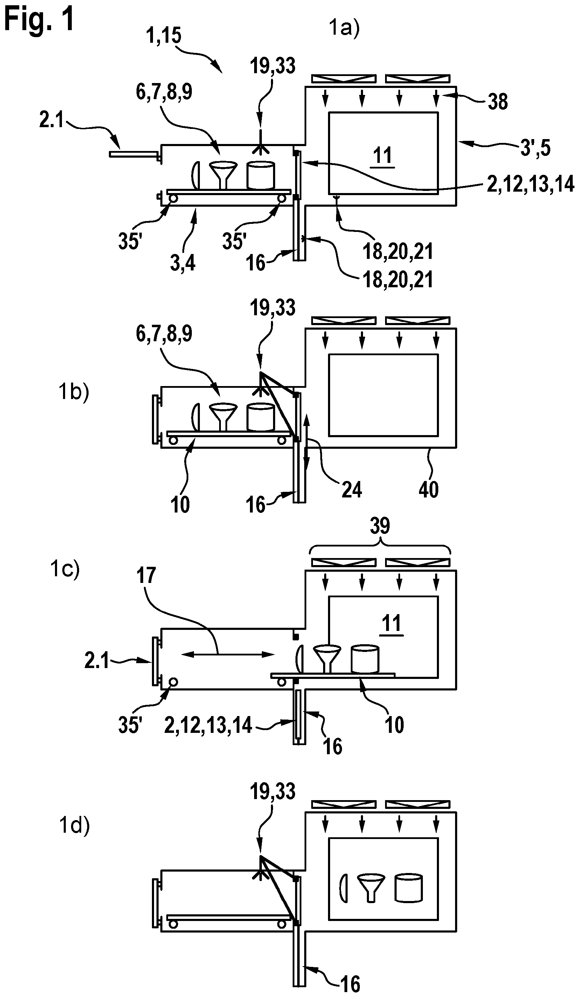

Figur 1 zeigt ein im Ganzen mit 1 bezeichnetes Transfersystem mit einer ersten kontrollierten Umgebung 3, die über eine Transferschnittstelle 2 mit einer zweiten kontrollierten Umgebung 3' gekoppelt ist. Die Chronologie der Verfahrensabläufe ergibt sich durch die Figuren und die nachstehende Figurenbeschreibung, ist jedoch nicht auf diese eingeschränkt.

In diesem Ausführungsbeispiel ist die erste kontrollierte Umgebung als Autoklav 4 und die zweite kontrollierte Umgebung als Isolator 5 ausgebildet. Der Autoklav 4 und der Isolator 5 sind in diesem Ausführungsbeispiel stationär ausgebildet.

Der Autoklav 4 ist ferner mit einer Beladeschnittstelle 2.1 ausgebildet, über die der Autoklav 4 mit wenigstens einem zu autoklavierenden Gegenstand 6, hier mehreren Gegenständen 6, beladen werden kann.

Bei den Gegenständen 6 handelt es sich um wenigstens eine Funktionseinheit 7, in diesem Ausführungsbeispiel um Bevorratungseinheiten 8 für Verbrauchsmaterial 9. Zum Transferieren der Gegenstände 6 ist eine über die Transferschnittstelle 2 bewegbare Zuführeinrichtung 10 ausgebildet, über die die Gegenstände 6 von der ersten kontrollierten Umgebung 3, hier dem Autoklaven 4, zu der zweiten kontrollierten Umgebung 3', hier dem Isolator 5, transferiert werden können. Die Zuführeinrichtung 10 kann in diesem Ausführungsbeispiel ausgezogen werden, da diese auf drehend gelagerten, hier ortsfesten, Rollen 35` aufgesetzt und durch diese entlang einer Transferbewegung 17 bewegbar ist. In weiteren, nicht gezeigten Ausführungsbeispiel ist die Zuführeinrichtung 10 mit daran gekoppelten Rollen 35` ausgebildet. Neben Rollen 35` sind auch weitere, eine Bewegung und/oder ein Gleiten der Zuführeinrichtung 10 bewirkende Elemente möglich.

Die Zuführeinrichtung 10 ist hierbei dazu ausgebildet, dass sich diese von der ersten kontrollierten Umgebung 3 bis zu einer in der zweiten kontrollierten Umgebung 3' ausgebildeten Verarbeitungsstation 11 zur Verarbeitung des Verbrauchsmaterials 9 bewegen, hier wie beschrieben verfahren, lässt.

Es kann somit zudem gesagt werden, dass in der ersten kontrollierten Umgebung 3 wenigstens eine Funktionseinheit 7, hier wenigstens eine Bevorratungseinheit 8 für Verbrauchsmaterial 9 und in der zweiten kontrollierten Umgebung 3' wenigstens eine Verarbeitungsstation 11 zur Verarbeitung des Verbrauchsmaterials 9 aufgenommen ist.

Der Isolator 5 wird ferner mit einem dem Erdschwerefeld entgegengesetzten Luftstrom 38 durchströmt, wobei der Luftstrom 38, bevor dieser in das Innere des Isolators 38 gelangt, über eine Filtereinheit 39 gefiltert wird, sodass verhindert werden kann, dass über den Luftstrom 38 Mikroorganismen, beispielsweise Partikel und/oder Verunreinigungen, insbesondere mikrobiologische Verunreinigungen, innerhalb des Isolators 5 gelangen. Die Richtung des Luftstroms 38 ist dabei derart gewählt, dass, selbst wenn Mikroorganismen in das Innere des Isolators 5 gelangen und/oder dort vorhanden sind, diese durch den Luftstrom 38 in Richtung eines unteren Abschnitts 40 des Isolators 5 "geleitet" werden.

Die Transferschnittstelle 2 ist im gezeigten Ausführungsbeispiel ferner als druckdichte Türe 12 ausgebildet, um den herrschenden Drücken während des Autoklavierens standzuhalten. Ferner bildet die Transferschnittstelle 2 eine dichte Begrenzung 13 des Isolators 5 und des Autoklaven 4.

Um verschieden große Gegenstände 6 von dem Autoklaven 4 zu dem Isolator 5 transferieren zu können und/oder um den Autoklaven 4 überhaupt mit unterschiedlich großen Gegenständen 6 beladen zu können, weist die Transferschnittstelle 2 einen unrunden Querschnitt 14 auf (nicht näher dargestellt). In einem nicht näher gezeigten Ausführungsbeispiel weist die Transferschnittstelle 2 einen rechteckigen Querschnitt 14 auf.

Im gezeigten Ausführungsbeispiel ist das Transfersystem 1 ein Rapid Transfer Port (RTP) 15.

Ferner ist in Figur 1 ein Verfahren zum Transferieren des Gegenstandes 6 zwischen der ersten kontrollierten Umgebung 3, also dem Autoklav 4, und der zweiten kontrollierten Umgebung 3', also dem Isolator 5, veranschaulicht.

Zunächst wird der Autoklav 4, insbesondere die Zuführeinrichtung 10 in dem Autoklav 4, mit den Gegenständen 6 über die Beladeschnittstelle 2.1 beladen (Figur 1, 1a)). Nachdem die Beladeschnittstelle 2.1 und/oder die Transferschnittstelle 2 geschlossen, evakuiert und/oder abgedichtet ist, folgt innerhalb des Isolators 5 zunächst eine Dekontamination 18 (Transferschnittstelle 2 geschlossen, siehe Figur 1, 1a)) und innerhalb des Autoklaven 4 eine Sterilisation 19 (Beladeschnittstelle 2.1 geschlossen, siehe Figur 1, 1b)), beispielsweise mittels heißem Dampf 33 unter hohem Druck. Anschließend wird die Transferschnittstelle 2 durch eine Klappbewegung in einen Bereich 16 bewegt, hier durch eine Klappbewegung 24, der außerhalb der Transferbewegung 17 der Zuführeinrichtung 10 liegt (Vgl. Figur 1, 1b)/1c)). Der Bereich 16 wurde dabei vor dem Transfer zusätzlich dekontaminiert (Figur 1, 1a)). Zur Dekontamination 18 wird hierbei ein Dekontaminationsmittel 20, in diesem Ausführungsbeispiel Wasserstoffperoxid 21, in den Isolator 5 unbeigemischt eingebracht und zerstäubt.

Anschließend kann die Zuführeinrichtung 10 von dem Autoklav 4 bis zur Verarbeitungsstation 11 im Isolator 5 bewegt und anschließend, nachdem die Gegenstände 6 entnommen wurden, wieder zurück zum Autoklav 4 bewegt (Figur 1, 1c)/1d)). Es folgt eine erneute Sterilisation 19 des Autoklaven 4 mittels Heißdampf 33, nachdem die Transferschnittstelle 2 wieder geschlossen wurde. Hierbei ist auch die Beladeschnittstelle 2.1 geschlossen.

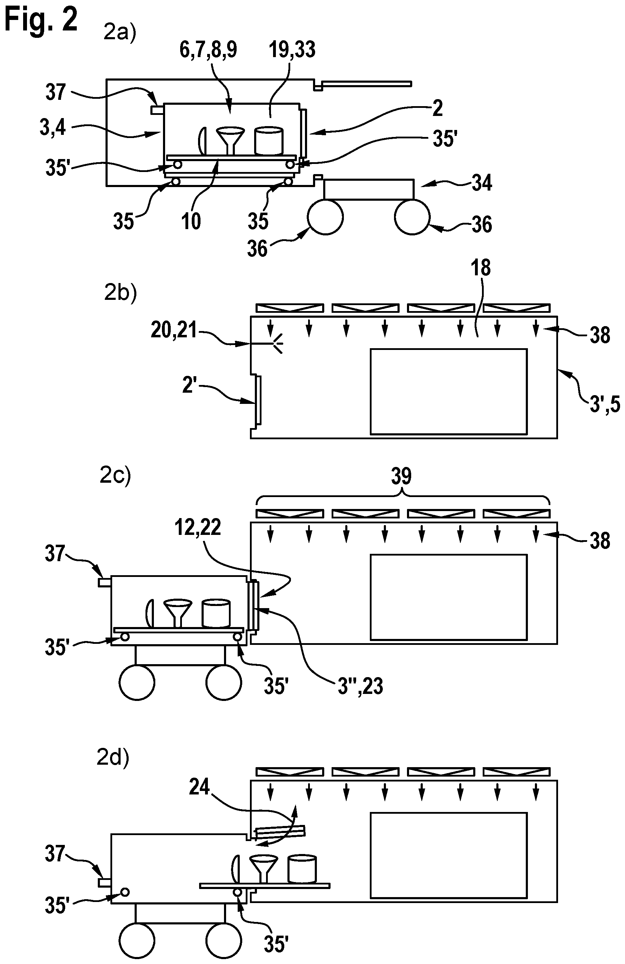

Figur 2 zeigt ein weiteres Transfersystem 1 mit ebenfalls einer ersten kontrollierten Umgebung 3 und einer zweiten kontrollierten Umgebung 3'. Auch in diesem Ausführungsbeispiel ist die erste kontrollierte Umgebung 3 als Autoklav 4 und die zweite kontrollierte Umgebung 3' als Isolator 5 ausgebildet, wobei ein Unterschied zum Ausführungsbeispiel gemäß Fig. 1 ist, dass der Autoklav 4 mobil und der Isolator 5 stationär ist. Der mobile Autoklaven 4 verfügt hierbei, wie gezeigt, über Rollen 35. In weiteren, nicht näher dargestellten Ausführungsbeispielen verfügt wenigstens eine der kontrollierten Umgebungen 3, 3' über weitere, mobilmachende und/oder reibungsmindernde Komponenten.

Funktionell und/oder konstruktiv zu dem vorhergehenden Ausführungsbeispiel gleichartige oder identische Bauteile und Funktionseinheiten sind mit denselben Bezugszeichen bezeichnet und nicht noch einmal gesondert beschrieben.

Zudem zeigt das Ausführungsbeispiel nach Figur 2, dass die Transferschnittstelle 2 des Autoklav 4 mit einer Transferschnittstelle 2' des Isolator 5 gekoppelt wird.

Somit veranschaulicht das Ausführungsbeispiel gemäß Figur 2 ein Verfahren zum Koppeln der ersten Transferschnittstelle 2 der ersten kontrollierten Umgebung 3, hier des Autoklaven 4, mit der zweiten Transferschnittstelle 2' der zweiten kontrollierten Umgebung 3', hier des Isolators 5.

In einem ersten Schritt (Figur 2, 2a)), hier vor dem Koppeln der kontrollierten Umgebungen 3, 3', werden Funktionseinheiten 7 beispielsweise die bereits erwähnten Funktionseinheiten 7, zunächst in dem Autoklaven 4 extern sterilisiert. Zuvor wurde die Zuführeinrichtung 10, und somit der Isolator 4, im Unterschied zum vorangegangenen Ausführungsbeispiel, über die Transferschnittstelle 2 mit den Funktionseinheiten 7 beladen, wodurch auf die Beladeschnittstelle 2.1 verzichtet werden kann.

Zeitgleich oder anschließend zu der Sterilisation 19 der Funktionseinheiten 7 mittels Heißdampf 33 innerhalb des Autoklaven 4, wird der Isolator 5 dekontaminiert. Zur Dekontamination 18 wird hierbei ebenfalls, wie im vorangehenden Ausführungsbeispiel bereits beschrieben, ein Dekontaminationsmittel 20, auch hier Wasserstoffperoxid 21, in den Isolator 5 ebenfalls unbeigemischt eingebracht und zerstäubt (siehe Figur 2, 2b)).

Während der Sterilisation 19 und Dekontamination 18 sind der Isolator 5 und der Autoklav 4 jeweils über die noch nicht miteinander gekoppelten Transferschnittstellen 2, 2' abgedichtet und/oder evakuiert (siehe Figur 2a) und 2b)).

Um den externen Autoklaven 4 transportieren zu können, wird dieser, bei noch geschlossener Transferschnittstelle 2, über die Rollen 35 auf eine Beförderungseinheit 34 bewegt (Figur 2, 2a)). Die Beförderungseinheit 34 verfügt wiederum über zwei Räder 36, sodass die gesamte Beförderungseinheit 34 ebenfalls mobil ist (Figur 2, 2a)).

Da sich die Zuführeinrichtung 10 innerhalb des Autoklaven 4 befindet, kann gesagt werden, dass wenigstens eine kontrollierte Umgebung 3, 3', hier der Autoklav 4, derart bewegbar ist, dass die Zuführeinrichtung 10 innerhalb der kontrollierten Umgebung 3 von der Beförderungseinheit 34 transportierbar ist. Zudem ist die Zuführeinrichtung 10, auf der die Funktionseinheiten 7 abgelegt sind, während des Transports des Autoklaven 4 zum Isolator 5 über die Beförderungseinheit 34 in einer gleichbleibenden Orientierung in dem Autoklaven 4 fixiert.

Anschließend werden die Transferschnittstellen 2, 2' des Autoklaven 4 und des Isolators 5 miteinander gekoppelt (Vgl. Figur 2, 2c)).

In diesem Ausführungsbeispiel bilden die Transferschnittstellen 2, 2' des Autoklaven 4 und des Isolators 5 im gekoppelten Zustand eine aus zwei Türen 12 bestehende druckdichte Doppeltüre 22, zwischen denen ein Zwischenbereich 23 gebildet ist (Vgl. Figur 2, 2c)). Dieser Zwischenbereich 23 ist in diesem Ausführungsbeispiel durch Vakuumerzeugung abdichtbar und/oder evakuierbar, sodass die als Türen 12 ausgebildeten Transferschnittstellen 2, 2' miteinander beweglich sind.

Es kann somit gesagt werden, dass die Transferschnittstellen 2, 2' kraft- und/oder formschlüssig miteinander koppelbar sind und dass die Transferschnittstellen 2, 2' den Zwischenbereich 23 begrenzen, in dem eine dritte kontrollierte Umgebung 3" ausbildbar ist (Vgl. Figur 2, 2c)).

Zum Transferieren der Funktionseinheiten 7 werden die Türen 12 anschließend, und somit nach der Sterilisation 19 des Autoklaven 4 und nach der Dekontamination 18 des Isolators 5, gemeinsam durch eine Klappbewegung 24 geöffnet (Vgl. Figur 2, 2d)). Die Zuführeinrichtung 10, auf der die Funktionseinheiten 7 positioniert sind, kann sodann von dem Autoklaven 4 bis zum Isolator 5 bewegt und die Funktionseinheiten 7 entnommen werden (Figur 2, 2d)).

Schließlich werden die Türen 12 gemeinsam geschlossen.

Der Autoklav 4 ist ferner mit einem Filter 37 ausgebildet, über den beispielsweise der Heißdampf 33 und/oder weitere sterilisierende und/oder dekontaminierende Fluide eingebracht werden kann/können. Ferner können über den Filter 37, beispielsweise beim Einbringen des Heißdampfs 33, Mikroorganismen, beispielsweise Partikel und/oder Verunreinigungen, insbesondere mikrobiologische Verunreinigungen, innerhalb des Heißdampfs 33 herausgefiltert werden, bevor diese in den Autoklaven 4 gelangen.

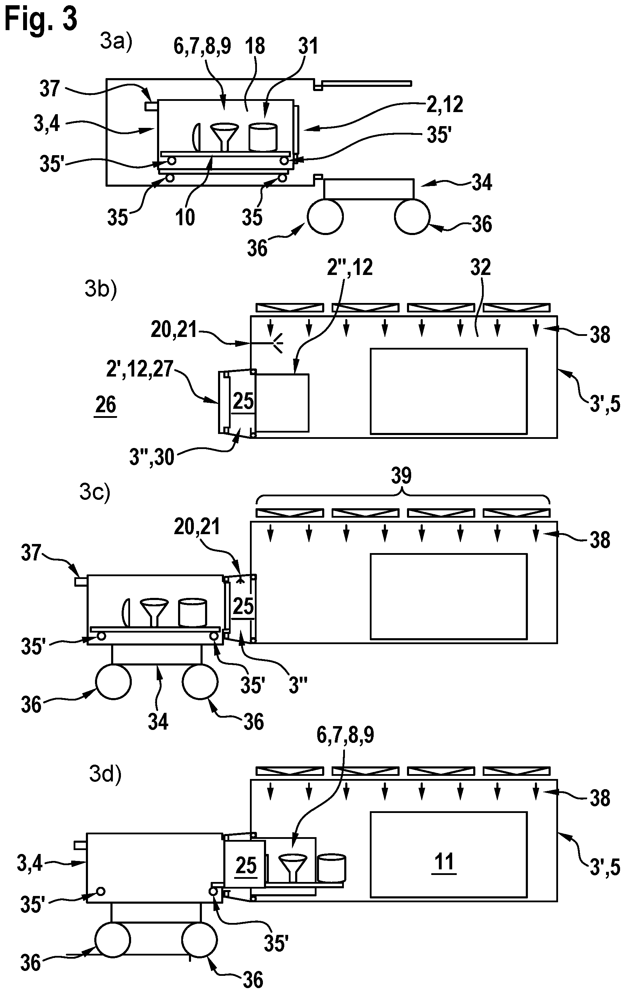

Figur 3 zeigt im Unterschied zu dem Ausführungsbeispiel gemäß Figur 2, dass der Isolator 5 zwei voneinander beabstandeten Transferschnittstellen 2', 2" aufweist, zwischen denen ein evakuierbarer und/oder abdichtbarer Zwischenraum 25 gebildet ist. Es kann somit gesagt werden, dass die Transferschnittstellen 2', 2" den Zwischenraum 25 begrenzen, in dem eine dritte kontrollierte Umgebung 3" gebildet ist.

Figur 3 veranschaulicht somit ein Verfahren zum Transferieren eines Gegenstandes 6, insbesondere von Funktionseinheiten 7, zwischen wenigstens einer ersten kontrollierten Umgebung 3 und einer zweiten kontrollierten Umgebung 3', wobei die erste kontrollierte Umgebung 3 und die zweite kontrollierte Umgebung 3' über einen Zwischenraum 25 miteinander koppelbar sind.

Ein Volumen 30 des Zwischenraums 25 ist hierbei kleiner als ein Volumen 31, 32 der ersten und zweiten kontrollierten Umgebung 3, 3'.

Auch in diesem Ausführungsbeispiel werden die Funktionseinheiten 7, wie bereits im Ausführungsbeispiel zu Figur 2 beschrieben, zunächst extern im Autoklaven 4 sterilisiert und anschließend über die Beförderungseinheit 34 zum Isolator 5 bewegt (Vgl. Figur 3, 3a)). Zeitgleich oder anschließend zur Sterilisation 19 wird der Isolator 5 gemeinsam mit dem Zwischenraum 25 dekontaminiert (Vgl. Figur 3, 3a)/3b)). Hierzu ist die dem Isolator 5 zugewandte Transferschnittstelle 2" geöffnet und die äußere, an eine äußere Umgebung 26 angrenzende Transferschnittstelle 2', geschlossen (Vgl. Figur 3, 3b)).

Die an die äußere Umgebung 26 angrenzende Transferschnittstelle 2' ist in diesem Ausführungsbeispiel zunächst von einem Blinddeckel 27 und die dem Isolator 5 zugewandte Transferschnittstelle 2" von einer druckdichten Türe 12 gebildet. Die Transferschnittstelle 2 des Autoklaven 4 ist ebenfalls von einer druckdichten Türe 12 gebildet.

In einem weiteren Ausführungsbeispiel ist die Transferschnittstelle 2' nicht von einem Blinddeckel 27, sondern ebenfalls von einer druckdichten Türe 12 gebildet.

Anschließend wird die dem Isolator 5 zugewandte Transferschnittstelle 2" geschlossen, der Blinddeckel 27 geöffnet und die Transferschnittstelle 2 des Autoklaven 4 über die Transferschnittstelle 2' mit dem Isolator 5 gekoppelt, sodass der Zwischenraum 25 evakuiert und/oder abgedichtet ist. Die Transferschnittstellen 2 und 2' begrenzen somit den Zwischenraum 25 (Vgl. Figur 3, 3c)).

Ist die Transferschnittstelle 2' von einer druckdichten Türe 12 gebildet, wird die Transferschnittstelle 2 des Autoklaven 4, wie im Ausführungsbeispiel nach Figur 2 beschrieben, mit der als druckdichten Türe 12 ausgebildeten, äußeren Transferschnittstelle 2' des Isolators gekoppelt, sodass die Transferschnittstellen 2, 2' zusätzlich den oder einen Zwischenbereich 23 begrenzen, der ebenfalls evakuierbar und/oder abdichtbar ist.

Anschließend wird der Zwischenraum 25 erneut dekontaminiert.

Es kann somit gesagt werden, dass vor und/oder während einer Dekontamination 18 des Zwischenraums 25 der Autoklav 4 sterilisiert und der Isolator 5 dekontaminiert wird.

Die Transferschnittstelle 2 und die mit dieser gekoppelte Transferschnittstelle 2' werden sodann gemeinsam und anschließend die Transferschnittstelle 2" geöffnet, sodass die Zuführeinrichtung 10 mitsamt Gegenstand 6 in bereits beschriebener Weise von dem Autoklaven 4, über den Zwischenraum 25 bis zur Verarbeitungsstation 11 im Isolator 5 bewegt werden kann (Vgl. Figur 3, 3d)). Die Transferschnittstellen 2, 2' und die Transferschnittstelle 2" werden somit nacheinander geöffnet. Eine umgekehrte Reihenfolge des Öffnens ist ebenfalls möglich. Somit kann auch zuerst die Transferschnittstelle 2" und anschließend die beiden miteinander gekoppelten Transferschnittstellen 2, 2' geöffnet werden. Die Türen 12 werden dabei seitlich durch eine Schiebebewegung geöffnet.

Anschließend werden die Türen 12 wieder geschlossen.

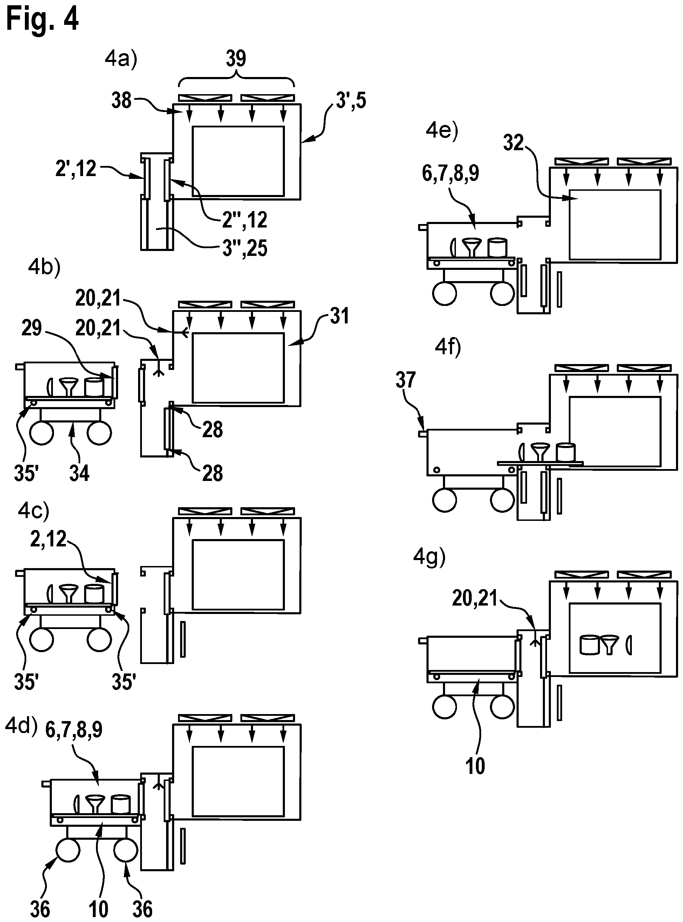

Figur 4 unterscheidet sich von dem Ausführungsbeispiel nach Figur 3 dadurch, dass die dem Isolator 5 zugewandte Transferschnittstelle 2" (Vgl. Figur 4, 4a)) zur gemeinsamen Dekontamination 18 des Zwischenraums 25 und des Isolators 5 zum Öffnen und/oder Verschließen des Zwischenraums 25 derart bewegt wird, dass sich diese innerhalb des Zwischenraums 25 befindet (Vgl. Figur 4, 4b)). Somit wird die gesamte dem Isolator 5 zugewandte Transferschnittstelle 2'', hier druckdichte Türe 12, mitsamt einer Dichtung 28 allseitig dekontaminiert (Vgl. Figur 4, 4b)).

Ferner ist der Zwischenraum 25 im Ausführungsbeispiel nach Figur 4 vor der Koppelung mit einer äußeren Begrenzung 29 des Autoklaven 4, die hier von der Transferschnittstelle 2 des Autoklaven 4 gebildet ist, nach außen offen (Vgl. Fig. 4, 4c)). Anschließend wird der Zwischenraum 25 durch die äußere Begrenzung 29 und somit durch die Transferschnittstelle 2, die hier ebenfalls als druckdichte Türe 12 ausgebildet ist, verschlossen (Vgl. Figur 4, 4d)). Es folgt eine Dekontamination 18 des Zwischenraums 25 (Vgl. Figur 4, 4d)).

Nach der Dekontamination 18 wird die dem Isolator 5 zugewandte Transferschnittstelle 2" geöffnet, sodass die Zuführeinrichtung 10 in bereits beschriebener Weise zunächst in den Isolator 5 und nach Übergabe der Gegenstände 6, hier Funktionseinheiten 7, wieder zurück zum Autoklaven 4 bewegt werden kann (Vgl. Figur 4, 4e)/4f)).

Die Türen 12 werden anschließend wieder geschlossen und der Zwischenraum 20 erneut dekontaminiert (Vgl. Figur 4, 4g)).

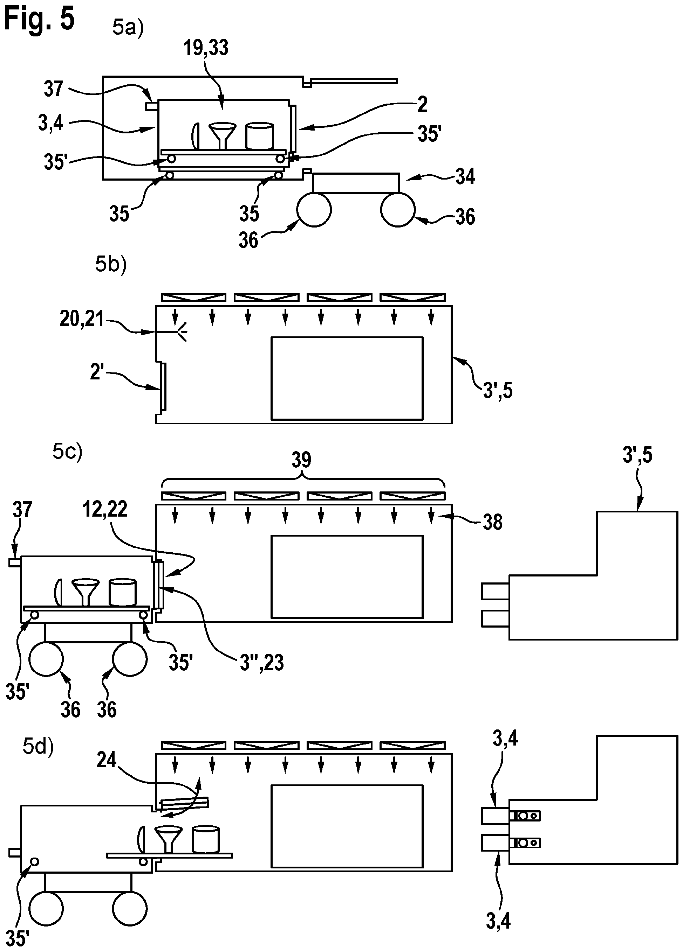

Figur 5 zeigt im Unterschied zu dem Ausführungsbeispiel nach Figur 2, dass zwei kontrollierte Umgebungen 3, 3" nebeneinander mit der kontrollierten Umgebung 3' gekoppelt sind. Die Verfahrensabläufe 2a) bis 2d) entsprechen dabei den Verfahrensabläufen 5a) bis 5d).

Die Erfindung schlägt somit allgemein ein Transfersystem 1 zum Transferieren von wenigstens einem Gegenstand 6 mit einer Zuführeinrichtung 10, die von einer ersten kontrollierten Umgebung 3, insbesondere einem Autoklav 4, zu einer zweiten kontrollierten Umgebung 3', insbesondere einem Isolator 5, über von der ersten und/oder zweiten kontrollierten Umgebung 3, 3' gebildeten, unrunden, insbesondere rechteckigen, Querschnitt 14 der Transferschnittstelle 2, 2', 2" beweglich ist, vor, wobei wenigstens zwei Transferschnittstellen 2, 2', 2" derart miteinander koppelbar sind, dass zwischen den Transferschnittstellen 2, 2', 2" ein Zwischenraum 25 und/oder ein Zwischenbereich 23 gebildet ist, wobei der Gegenstand 6 nach einer Dekontamination 18 und/oder Sterilisation 19 der ersten und/oder zweiten kontrollierten Umgebung 3, 3' transferiert wird.

### Bezugszeichenliste

- 1: Transfersystem
- 2: Transferschnittstelle
- 2': Transferschnittstelle
- 2": Transferschnittstelle
- 2.1: Beladeschnittstelle
- 3: erste kontrollierte Umgebung
- 3': zweite kontrollierte Umgebung
- 3": dritte kontrollierte Umgebung
- 4: Autoklav
- 5: Isolator
- 6: Gegenstand
- 7: Funktionseinheit
- 8: Bevorratungseinheit
- 9: Verbrauchsmaterial
- 10: Zuführeinrichtung
- 11: Verarbeitungsstation
- 12: Türe
- 13: dichte Begrenzung
- 14: Querschnitt
- 15: Rapid Transfer Port (RTP)
- 16: Bereich
- 17: Transferbewegung
- 18: Dekontamination
- 19: Sterilisation
- 20: Dekontaminationsmittel
- 21: Wasserstoffperoxid
- 22: Doppeltüre
- 23: Zwischenbereich
- 24: Klappbewegung
- 25: Zwischenraum
- 26: äußere Umgebung
- 27: Blinddeckel
- 28: Dichtung
- 29: äußere Begrenzung
- 30: Volumen
- 31: Volumen erste kontrollierte Umgebung
- 32: Volumen zweite kontrollierte Umgebung
- 33: heißer Dampf
- 34: Beförderungseinheit
- 35: Rollen
- 35`: Rollen
- 36: Rad
- 37: Filter (Fig. 2)
- 38: Luftstrom
- 39: Filtereinheit
- 40: unterer Abschnitt

## Patentansprüche

1. Transfersystem (1) mit wenigstens einer ersten Transferschnittstelle (2) einer ersten kontrollierten Umgebung (3), die mit wenigstens einer zweiten Transferschnittstelle (2') wenigstens einer zweiten kontrollierten Umgebung (3') koppelbar ist, und einer über die Transferschnittstellen (2, 2') von der ersten kontrollierten Umgebung (3) zu der zweiten kontrollierten Umgebung (3') bewegbaren Zuführeinrichtung (10), **dadurch gekennzeichnet, dass** die Transferschnittstellen (2, 2') einen unrunden, vorzugsweise rechteckigen, Querschnitt (14) aufweisen.

2. Transfersystem (1) nach Anspruch 1, **dadurch gekennzeichnet, dass** wenigstens eine kontrollierte Umgebung (3, 3') ein Isolator (5) und/oder ein Autoklav (4) ist und/oder dass wenigstens eine kontrollierte Umgebung (3, 3') mobil oder stationär ist.

3. Transfersystem nach einem der vorangehenden Ansprüche, **dadurch gekennzeichnet, dass** wenigstens eine kontrollierte Umgebung (3, 3') derart bewegbar ist, dass die Zuführeinrichtung (10) innerhalb der kontrollierten Umgebung (3, 3') von einer Beförderungseinheit (34) transportierbar und/oder in einer Orientierung in der kontrollierten Umgebung (3, 3') fixiert ist.

4. Transfersystem (1) nach einem der vorangehenden Ansprüche, **dadurch gekennzeichnet, dass** die Transferschnittstellen (2, 2'), insbesondere druckdichte, Türen (12) haben und/oder dass ein Zwischenbereich (23) und/oder ein Zwischenraum (25) zwischen den Transferschnittstellen (2, 2'), insbesondere den Türen (8), abdichtbar und/oder evakuierbar ist, insbesondere wobei die Türen (12) bei evakuiertem und/oder abgedichteten Zwischenbereich (23) miteinander beweglich sind.

5. Transfersystem (1) nach einem der vorangehenden Ansprüche, **dadurch gekennzeichnet, dass** das Transfersystem (1) ein Rapid Transfer Port (15) ist und/oder dass die Transferschnittstellen (2, 2') kraft- und/oder formschlüssig miteinander koppelbar sind.

6. Transfersystem (1) nach einem der vorangehenden Ansprüche, **dadurch gekennzeichnet, dass** die Transferschnittstellen (2, 2') der kontrollierten Umgebungen (3, 3') jeweils eine dichte Begrenzung (13) der kontrollierten Umgebung (3, 3') bilden und/oder dass in der ersten kontrollierten Umgebung (3) wenigstens eine Funktionseinheit (7), insbesondere eine Bevorratungseinheit (8) für Verbrauchsmaterial (9), und in der zweiten kontrollierten Umgebung (3') wenigstens eine Verarbeitungsstation (11) für Verbrauchsmaterial (9) aufgenommen ist.

7. Transfersystem (1) nach dem Oberbegriff von Anspruch 1 oder nach einem der vorangehenden Ansprüche, **dadurch gekennzeichnet, dass** die Transferschnittstellen (2, 2') den oder einen Zwischenbereich (23) und/oder den oder einen Zwischenraum (25) begrenzen, in dem eine dritte kontrollierte Umgebung (3") ausbildbar ist.

8. Transfersystem (1) nach einem der vorangehenden Ansprüche, **dadurch gekennzeichnet, dass** der Zwischenbereich (23) und/oder der Zwischenraum (25) abgedichtet und/oder evakuiert ist und/oder dass der Zwischenbereich (23) mit Türen (12) der Transferschnittstellen (2, 2') mitbewegbar ist.

9. Transfersystem (1) nach einem der vorangehenden Ansprüche, **dadurch gekennzeichnet, dass** die Transferschnittstellen (2, 2') miteinander koppelbar sind, insbesondere zum gemeinsamen Öffnen und/oder Schließen.

10. Transfersystem (1) nach einem der vorangehenden Ansprüche, **dadurch gekennzeichnet, dass** wenigstens eine Transferschnittstelle (2, 2') von einem Blinddeckel (27) gebildet ist und/oder dass ein Volumen (30) des Zwischenraums (25) und/oder des Zwischenbereichs (23) kleiner ist als ein Volumen der ersten und/oder zweiten kontrollierten Umgebung (31, 32).

11. Verfahren zum Transferieren eines Gegenstandes (6), insbesondere von Funktionseinheiten (7), zwischen wenigstens einer ersten kontrollierten Umgebung (3) und einer zweiten kontrollierten Umgebung (3'), wobei die erste kontrollierte Umgebung (3) und die zweite kontrollierte Umgebung (3') über wenigstens eine Transferschnittstelle (2, 2') miteinander gekoppelt werden, wobei zunächst die erste und die zweite kontrollierte Umgebung (3, 3') dekontaminiert und/oder sterilisiert werden, **dadurch gekennzeichnet, dass** nach der Dekontamination (18) und/oder Sterilisation (19) eine Zuführeinrichtung (10) eines Transfersystems (1), insbesondere nach einem der vorangehenden Ansprüche, von der ersten kontrollierten Umgebung (3) zur zweiten kontrollierten Umgebung (3') bewegt wird.

12. Verfahren nach einem der vorangehenden Ansprüche, **dadurch gekennzeichnet, dass** wenigstens eine Transferschnittstelle (2, 2'), insbesondere durch eine Klappbewegung (24), in einen Bereich (16) bewegt wird, der außerhalb einer Transferbewegung (17) der Zuführeinrichtung (10) liegt, wobei der Bereich (16) ebenfalls dekontaminiert wird.

13. Verfahren zum Koppeln wenigstens einer ersten Transferschnittstelle (2) einer ersten kontrollierten Umgebung (3) mit wenigstens einer zweiten Transferschnittstelle (2') wenigstens einer zweiten kontrollierten Umgebung (3') mit einem Transfersystem (1), insbesondere nach einem der vorangehenden Ansprüche, **dadurch gekennzeichnet, dass** die Transferschnittstellen (2, 2') einen Zwischenraum (25) und/oder einen Zwischenbereich (23) begrenzen, der vor einem Öffnen wenigstens einer Transferschnittstelle (2, 2') wenigstens einer kontrollierten Umgebung (3, 3') abgedichtet und/oder evakuiert wird.

14. Verfahren nach dem vorangehenden Anspruch, **dadurch gekennzeichnet, dass** vor und/oder während einer Dekontamination (18) und/oder einer Sterilisation (19) des Zwischenraums (25) und/oder des Zwischenbereichs (23) wenigstens eine kontrollierte Umgebung (3, 3') sterilisiert und/oder dekontaminiert wird.

15. Verfahren nach einem der vorangehenden Ansprüche, **dadurch gekennzeichnet, dass** der Zwischenraum (25) und/oder der Zwischenbereich (23) während der Dekontamination (18) und/oder der Sterilisation (19) abgedichtet und/oder evakuiert wird.

16. Verfahren nach einem der vorangehenden Ansprüche, **dadurch gekennzeichnet, dass** die Transferschnittstellen (2, 2') von, insbesondere druckdichten, Türen (12) gebildet sind, die vor, nach oder während der Dekontamination (18) und/oder Sterilisation (19) wenigstens einer kontrollierten Umgebung (3, 3'), vorzugsweise durch eine Klappbewegung (24) und/oder eine Schiebebewegung, geöffnet werden.

17. Verfahren zum Transferieren eines Gegenstandes (6), insbesondere von Funktionseinheiten (7), zwischen wenigstens einer ersten kontrollierten Umgebung (3) und einer zweiten kontrollierten Umgebung (3'), wobei die erste kontrollierte Umgebung (3) und die zweite kontrollierte Umgebung (3') über einen Zwischenraum (25) miteinander koppelbar sind, **dadurch gekennzeichnet, dass** der Zwischenraum (25) vor der Koppelung nach außen offen ist und der Zwischenraum (25) durch eine Begrenzung (29) der ersten kontrollierten Umgebung (3) verschlossen wird, dass der verschlossene Zwischenraum (25) dekontaminiert wird und dass nach der Dekontamination (18) zumindest die erste kontrollierte Umgebung (3) zum Zwischenraum (25) geöffnet wird.

18. Verfahren nach dem vorangehenden Anspruch, **dadurch gekennzeichnet, dass** die Koppelung über wenigstens eine Transferschnittstelle (2, 2', 2"), insbesondere druckdichte Tür (12), erfolgt und/oder dass zum Verschließen und/oder Öffnen des Zwischenraumes (25) wenigstens eine Transferschnittstelle (2, 2', 2"), insbesondere druckdichte Tür (12), derart bewegt wird, dass sich diese, zumindest teilweise, innerhalb des Zwischenraumes (25) befindet.

19. Verfahren nach dem Oberbegriff von Anspruch 16 oder nach einem der vorangehenden Ansprüche, **dadurch gekennzeichnet, dass** zur Dekontamination (18) ein Dekontaminationsmittel (20) in den Zwischenraum (25) und/oder eine erste und/oder zweite kontrollierte Umgebung (3, 3') unbeigemischt eingebracht und zerstäubt wird.
